# EUROPEAN PATENT SPECIFICATION

(11) **EP 1 810 037 B1**
(45) Date of publication and mention of the grant of the patent: **21.07.2010**
(21) Application number: 05797120.2
(22) Date of filing: 20.10.2005
(51) Int. Cl.: G01N 33/74

(54) **ASSESSMENT OF BIOLOGICAL ACTIVITY OF HEPATOCYTE GROWTH FACTOR (HGF)**
BEURTEILUNG DER BIOLOGISCHEN AKTIVITÄT VON HGF (HEPATOCYTE GROWTH FACTOR)
EVALUATION DE L'ACTIVITE BIOLOGIQUE DU FACTEUR DE CROISSANCE DES HEPATOCYTES (HGF)

(30) Priority: 20.10.2004 US 620610 P
(43) Date of publication of application: 25.07.2007
(73) Proprietor: PEAS Institut AB, 587 23 Linköping (SE)
(72) Inventor: NAYERI, Fariba, 58758 Linköping (SE)
(74) Representative: Sörby, Lennart
(86) International application number: PCT/SE2005/001583
(87) International publication number: WO 2006/043892

(56) References cited:
- EP-A- 1 636 593
- EP-A- 1 694 700
- WO-A-02/17964
- WO-A-2004/078778
- WO-A1-02/17964
- WO-A2-03/016781
- HORI TAKESHI ET AL: "Activation of hepatocyte growth factor in monkey stomach following gastric mucosal injury." JOURNAL OF GASTROENTEROLOGY 2004, vol. 39, no. 2, February 2004 (2004-02), pages 133-139, XP002491280 ISSN: 0944-1174
- KUNO Y ET AL: "POSSIBLE INVOLVEMENT OF NEUTROPHIL ELASTASE IN IMPAIRED MUCOSAL REPAIR IN PATIENTS WITH ULCERATIVE COLITIS" JOURNAL OF GASTROENTEROLOGY, SPRINGER VERLAG, TOKYO, JP, vol. 37, no. SUPPLEMENT 14, 1 January 2002 (2002-01-01), pages 22-32, XP008044736 ISSN: 1340-9077
- ZIONCHECK T F ET AL: "Sulfated oligosaccharides promote hepatocyte growth factor association and govern its mitogenic activity" JOURNAL OF BIOLOGICAL CHEMISTRY, AMERICAN SOCIETY OF BIOLOCHEMICAL BIOLOGISTS, BIRMINGHAM,; US, vol. 270, no. 28, 1 January 1995 (1995-01-01), pages 16871-16878, XP002303465 ISSN: 0021-9258
- MERKULOVA-RAINON TATYANA ET AL: "The N-terminal domain of hepatocyte growth factor inhibits the angiogenic behavior of endothelial cells independently from binding to the c-met receptor." THE JOURNAL OF BIOLOGICAL CHEMISTRY 26 SEP 2003, vol. 278, no. 39, 26 September 2003 (2003-09-26), pages 37400-37408, XP002491281 ISSN: 0021-9258
- TAKAHASHI M. ET AL: 'Hepatocyte Growth Factor is the Most Potent Endogenous Stimulant of Rabbit Gastric Epithelial Cell Proliferation and Migration in Primary Culture' J. CLIN. INVEST. vol. 95, May 1995, pages 1994 - 2003, XP002995311
- NAYERI F. ET AL: 'Hepatocyte growth factor (HGF) in fecal samples: rapid detection by surface plasmon resonance' BMX GASTROENTEROLOGY, BIOMED CENTRAL 2005, pages 1 - 8, XP002995312
- KOBAYASHI ET AL.: "Hepatocyte grouth factor specifically binds to sulfoglycolipids" THE JOURNAL OF BIOLOGICAL CHEMISTRY, vol. 269, no. 13, 1994, pages 9817-9821,

## Description

### FIELD OF THE INVENTION

The present invention relates to methods for evaluation of biological activity of hepatocyte growth factor (HGF). The methods can be used for quality control of HGF after batch production, e.g. by recombinant techniques and also for evaluation of HGF preparations prior to administration to a patient in need of such treatment. The evaluation methods can also be used to improve diagnosis of HGF-related diseases and subsequent therapy choices. The present invention therefore also relates to methods for evaluating the activity of endogenous HGF at the site of injury in ulcer secrete, urine, feces, saliva, pleura in order to assess the roll of HGF and determine if application of exogenous HGF to the injured area is required.

### BACKGROUND OF THE INVENTION

Hepatocyte growth factor (HGF) is a growth factor, that is secreted in response to cell damage and appears to be important for the regeneration of certain organs and healing of wounds (Arakaki N et al. Hepatology 221, 1995). It is a heterodimer, with heavy and light chains of approximately 60 and 30 kDa respectively, first synthesized as an inactive precursor (Miyazawa K et al. J Biol Chem 268, 1994). The precursor is cleaved to an active protein in the damaged organ by a specific activator (Naka D et al. J Biol Chem 276, 1992, Nishizaki T et al. J Am Coll Surg 181, 1995). HGF acts paracrinally as well as endocrinally (Yanagita K. et al. Biochem Biophys Res Commun 182, 1992, Kono S et al. Biochem Biophys Res Commun 186, 1992). The target cells of HGF are fully developed epithelial cells (Matsumoto K and Nakamura T. J Gastroenterology Hepatology 6, 1991). HGF is produced and is present in high concentrations at sites of organ damage (Nayeri F et al. Scand J Infect Dis, 34, 2002). We have previously studied the systemic and local production of HGF in various infectious diseases and have observed high serum HGF concentrations during acute infectious diseases such as gastroenteritis (Nayeri F et al. Scand J Infect Dis, 34, 2002). Simultaneous with enhanced systemic production of HGF, we found high HGF concentrations in cerebrospinal fluid during meningitis (Nayeri F et al. J Infect Dis, 181, 2000). Raised HGF concentrations in exhaled breath condensate (Nayeri F, et al. Respir Med, 96, 2002) in patients with pneumonia, which had no correlation to serum levels of HGF, indicated a local production of HGF during pneumonia. Furthermore we have studied the stability of HGF in serum (Nayeri F, et al, CYTOLIKE, 2002) and found that HGF was very stable in samples (serum/plasma, feces, exhaled breath condensate) and several freeze-thaw cycles, different buffers or several years of storage at -20 °C did not affect HGF concentrations significantly.
HGF is a multifunctional growth factor and the regenerative properties of HGF have been studied in animal models in liver (Ishiki et al. 1992), kidneys (Igawa et al. 1993), lungs (Yanagita et al, 1993) and during sepsis (Kondo et al. 1999). It has been suggested that the mitogenic effect of HGF on the target cells is not sufficient to result in tissue regeneration and growth. Other functions of HGF such as motogenic and morphogenic effects are necessary to complete the regeneration process (Montesano et al., 1991). It is postulated that the truncated species of HGF may function as antagonists to growth promoting effects of HGF at the final stages of tissue regeneration and repair (Chan et al., 1991).

We have previously shown that exogenous HGF administration could heal chronic, difficult to treat, leg ulcers in patients (Nayeri et al, Dermatological Treatment, 2002). During a recent double-blind study we have used a commercial recombinant HGF that showed later to be defective. Using this defective component in the study preparation did not cause significant healing in ulcers. Some of these patients (n=4) received active recombinant HGF after one year and showed a significant healing in their ulcers within the first week (Table 1).
Due to therapy potential of HGF in treatment of infectious diseases antibiotics (EP 01 963 646.3) as well as future indications it is very important to assess the biological activity of HGF to be used as therapeutic component.

Further prior art is disclosed in the following documents.

Hori et al (J Gastroenterol 2004, 39, 2, 133-139) is a study of the conversion of pro-HGF to mature HGF in response to induced gastric mucosal injury. The authors detect mature HGF with a known ELISA. The authors measure the presence of mature HGF, but not the biological activity of HGF.

Kuno et al (J Gastroenterol 2002, 37, suppl 14, 22-32) is a study of the expression of HGF in colonic mucosal tissues, see Background/Methods. A commercially available ELISA is used, see page 24 "Assay for HGF, KGF and HGF-β". Again, this study measures the presence, but not the biological activity, of HGF.

Zioncheck et al (J Biol Chem, 270, 28, 16871-16878) measures the biological activity of rhHGF by measuring [³H]thymidine incorporation into rat hepatocyte DNA, see page 16872 section "HGF Bioassay". This document does not show measurement of the affinity of HGF to dextran in order to determine the biological activity of said HGF.

WO02/17964 relates to the combined use and synergistic effects of HGF and antibiotics and , to a kit for determining the presence of HGF in excrement. Also this document is concerned with the presence of HGF and presumes that all HGF is biologically active.

Merkulova-Rainon et al (J Biol Chem, 278, 39, 37400-37408) discloses binding studies of HGF and HGF-domains and c-met (page 37402, left column, last paragraph). WO2004/078778 disclose different c-met receptor densities bound on the surface of a CMS chip. D6 does not correlate binding of HGF to c-met with the biological activity of HGF.

EP 1,636,593 discloses the expression and purification of HGF β proteins and the binding of HGF β to Met ECD domain (page 73-73) and to Met-IgG fusion protein (page 75 lines 4-24), and of HGF mutants to Met-IgG fusion protein (page 75 lines 25-35). No correlation between binding/affmity and biological activity is made.

Kobayashi et al (J Biol Chem, 269, 13, 9817-9821) studied binding of HGF to various glyco lipids.

The present invention describes in part, a method for evaluation of HGF activity in **recombinant preparations**, as well as in **biological products.**

### SUMMARY OF THE INVENTION

It is an object of the present invention to provide a method of assessment of biological activity of recombinant HGF as well as HGF detected in body fluids.

The present invention relates to an in vitro method for assessment of biological activity of HGF. In the evaluation of biological activity the interactions with dextran, are used. For studies of these interactions a number of methods are available and well known to a person of skill in the art. In a preferred embodiment of the invention an instrument provided by BIACORE (Uppsala, Sweden) is used. The technique is based on the Surface Plasmon Resonance method and is very effective in that a number of interactions can be studied in parallel. However, the methods according to the invention are of course not limited to a specific instrument even if the description for clarity is focused on this embodiment.

### DETAILED DESCRIPTION OF THE INVENTION

### Mutants of HGF:

Hepatocyte growth factor (HGF) is a large protein with a molecular weigh of about 90 kDa. When activated the protein is cleaved to alfa and beta chains. The previous studies have shown that deletion of the N-terminal hairpin domain, the first kringle domain, the second kringle domain or the serine proteinase domain in the HGF molecule results in a total loss of biological activities (Matsumoto et al., 1991b). Deletion of either the α-chain or the β-chain in the HGF molecule results in a complete loss of activity in HGF (Matsumoto et al., 1991). The *c-met* receptor-binding domain has been identified on the NH2-terminal of the α-chain of HGF (Mizuno et al., 1994). A three-dimensional model of the 27-residue hairpin loop, which plays an important role in receptor binding and the activity of HGF, has also been obtained by protein engineering and nuclear magnetic resonance (NMR) techniques (Zhou et al., 1998). The sequence features of HGF together with the results of domain deletion experiments and the availability of 3D models of individual domains have led to several studies in which specific residues or clusters of residues have been substituted in order to clarify their role in HGF activity (Chirgadze et al., 1998). Lokker et al. (1994) have studied the residues critical for receptor binding within the N domain. A number of individual and group mutations have so far failed to identify residues critical for receptor binding within the hairpin loop itself. However they found that a cluster of residues located at the C terminus of the N domain (His-114, Glu-115, Asp-171) were involved in both receptor binding and the biological activity of HGF (Lokker et al., 1994). They have also shown that substitution of at least seven amino acids in the first kringle (Arg-197, Glu-189, Tyr-198, Glu-195, Asp-171, Gln-173 and Ser-161) has a clear effect on receptor binding and the biological activity of HGF. The greatest effect is seen with substitution of Arg-197 and Glu-159, which reduce receptor binding and biological activity more than fifty fold (Lokker et al., 1994). Several point mutations in the serine proteinase domain of HGF (in which residues Gln-534 and Tyr-673 were reverted to the His and Ser residues of active serine proteinase) did not affect receptor binding but markedly decreased biological activity. A similar result was obtained with Val692Ser mutation (Lokker et al., 1992). Mutation of critical residues at the trypsin-like cleavage site, located between the C-terminal kringle (kringle 4) and the serine proteinase domain, abolished conversion of the single chain precursor form of HGF (pro-HGF). The resulting single chain protein retained receptor binding activity but lacked the biological activity of HGF on the target cells (Lokker et al., 1992; Naka et al., 1992; Gak et al., 1992).

### Importance of the assessment of biological activity of HGF

In a previous pilot study HGF in gel form was locally applied, once daily for seven days, to 15 of 19 chronic leg ulcers in eleven elderly patients. All patients had previously been treated by conventional methods and their leg ulcers were in rather stable condition for a duration of between one and fourteen years. Any signs of allergy, discomfort or pain were reported daily. Microcirculation perfusion in the ulcers, compared to the intact contiguous skin, was determined by laser Doppler at the beginning of the study, after one week and again after three months (in 7 patients). Ulcer size and characteristics were also documented. We observed that microcirculatory perfusion, which might reflect the angiogenic effect of HGF, was statistically significantly correlated (r= 0.94, p<0.002) to ulcer area reduction in the treated ulcers. Excellent (84-100% area reduction) or partial healing (58-59%) was seen in 8/11 patients.

To evaluate the positive effects of hepatocyte growth factor on chronic leg ulcers observed during this pilot study, a double-blinded placebo-controlled study on 20 patients using a commercial recombinant HGF in one week was initiated. The study was interrupted after inclusion of twelve patients when SDS-PAGE and western blot revealed that the protein used was defective (only one chain of about 60-67 kDa was found). Immunohistochemistry staining of skin biopsies from the ulcer area revealed a significantly higher expression of c-met receptor in the patients (n=12) compared to normal healthy controls (n=10, p<0.001). Although a tendency to decrease in c-met expression was observed in patients who had received the preparation containing commercial recombinant HGF (Group 1, n=6) (might indicate interaction of protein with c-met receptor), the differences between this group and the group who received sodium chloride preparation (Group 2, n=6) was not significant (Table 1). Enhanced capillary proliferation in Group 1 was observed (non-significant), but only in one case a dramatic clinical improvement was seen. Nevertheless, three cases in Group 1 and two in Group 2 were healed within 9 months. The other seven patients were offered treatment with biologically active recombinant HGF, and four (two in each group) accepted (Group 3). Improvement in the ulcer situation was observed in all of these initially unsuccessful cases within the first week of treatment. The c-met expression at the ulcer area was determined in two patients and was decreased after one week of treatment in both cases.

We concluded that biologically active HGF had positive effects on healing of chronic leg ulcers not attributed to placebo effects. The properties of the defective and active recombinant HGF were investigated in order to characterize the biologically active forms of HGF that have therapeutic effect in patients. As mentioned previously the mitogenic and motogenic effects of HGF are needed for an effective healing process.

### BRIEF DESCRIPTION OF THE DRAWINGS

**Figure 1****:** Restitution in control (plain) and active HGF treated cells within 24 and 48 hours after injury.
**Figure 2****:** Different concentration of HGF (1, 5 and 10 ng/ml) and differences in restitution within 24 hours.
**Figure 3****:** Localisation of c-Met receptor in CCL-53.1 cells by Immunofluorescence technique. As it has been shown there are not differences between biologically active HGF treated (A) and control (B) in presentation of receptor by Immunofluorescence. But the reduction of injured bare area is clearly visible in (A) compared to (B).
**Figure 4****:** The migration assay. The picture on the right shows migration of cells after addition of biologically active HGF and the picture on the left shows control cells. The arrows show the edge of round cover glass.
**Figure 5****: :** Confocal microscopy and 3D look-through projections comparing control (left) and active HGF-treated cells (right) after phalludin labelling of actin structures. Please note the difference in the cell wall diameter between the two groups.
**Figure 6****:** The morphological differences between biologically inactive (left) and control cells (right).
**Figure 7****:** SPR response of biologically active HGF before (the first signal on left) and after addition of dextran (0.05%) (the second signal). The regeneration pulse is observed after each signal.
**Figure 8****:** Historam showing the differences between the SPR signal from biologically active HGF and inactive HGF.
**Figure 9****:** Shows the difference between hair growth in mice receiving biologically active HGF subcutaneously for 5 days (on left and right side) and the control mouse receiving sterile sodium chloride injections subcutaneously for 5 days (in the middle). The picture is taken on day 12 after first injection.

### EXAMPLES

In the present invention the following critera have been adopted to define HGF with a biological activity suitable for use in therapeutic treatments. Using the methods of the present invention biologically active HGF
- should cause at least 50% decrease in damaged area in the CCL-53.1 model within 18 hours;
- should have an equal affinity to (50ug/mL) immobilized monoclonal and polyclonal anti-HGF antibodies as well as (20ug/mL) c-met protooncogen HGF receptor in a SPR based method;
- should have an affinity lower than the affinity to monoclonal anti-HGF antibody and higher than the negative control to dextran in a SPR based method. This affinity should disappear when dextran is added to the samples;
- should cause a twice as rapid hair growth in shaved skin areas of mice judged by expression of PCNA in skin biopsies obtained from hairless areas one week after treatment by HGF (at least 100ng/mouse/day for 5 days) determined by immunohistochemistry staining or by determining the time for the animal to show a complete hair growth in the hairless area (less than 15 days).

The following examples are intended to further illustrate the embodiments of the invention and are not intended to limit the scope of the invention in any way.

### EXAMPLE 1: Effects of hepatocyte growth factor in accelerating restitution of damaged mouse melanocyte cells in vitro

The following materials and conditions were used.

**Cells:** CCL-53.1 adherent epithelial cells derived from mouse skin, melanocytes, melanoma were obtained from American Tissue Cell Collection (ATCC, Manassas, Virginia, USA) and were used at passages 3-32.
**Growth factors:** Recombinant Hepatocyte growth factor (HGF) was a kind gift from professor T Nakamura, Osaka, Japan. HGF was also obtained commercially (R D Systems, Lot: QF031062), Fibroblast growth factor (FGF, R D Systems, Lot: EX164011), Platelett-derived growth factor (PDGF, R D Systems, Lot:D332071), Keratinocyte growth factor (KGF, R D Systems, Lot No:J0164021), Epidermal growth factor (EGF, R D Systems, Lot:HLM 1140311).

As the recombinant HGF obtained from professor Nakamura was shown to be very effective in treatment of chronic ulcers in patients (Example 4) it has been used herein as a positive control for biologically active HGF.

**Cell culture:** CC1-53.1 cells were grown in Kaighn's modification of Ham's F-12K medium (ATCC) supplemented with 15% horse serum and 2.5% fetal bovine serum (Sigma-Aldrich Stockholm, Sweden), in an atmosphere of 5% CO₂ and 95% air at 37°C. After the cells reached confluence, they were separated with non-enzymatic cell dissociation solution (1x) (Sigma-Aldrich) and suspended in F-12K medium with 15% horse serum and 2.5% fetal bovine serum and inoculated on a 24-well culture plate (Nunc Brand Products, Roskilde, Denmark) and cultured under the same conditions for 24-48 hours until they reached confluence.

**Serum-free medium:** Confluent cultures of CC1-53.1 cells grown in 75-cm² tissue culture flasks (Nuncolone) were washed with phosphate buffer sulotion (Sigma-Aldrich) and separated with non-enzymatic cell dissociation solution and resuspended in serum-free medium Nutridoma-NS medium (Roche Diagnostics Scandinavia AB, Bromma, Sweden) and inoculated on a 24-well culture plate and cultured under the conditions described above for 24-48 hours until they reached confluence.

**Wounding assay:** The confluent monolayer was scraped by a sterile steel device. Detached cells were washed with PBS and fresh medium was added to the wells. The area of the square not covered by cells (mm²) was measured by microscope (Olympus) and documented in each well. HGF (1, 5, 10 ng/ml) were then added to the cells and incubated at 37°C in a humidified atmosphere containing 5% CO₂. After 24 and 48 hours the area not covered by monolayer was measured again and documented. Experiments were performed in triplicate. The median of triplicates was used for calculation of mean values of thirteen independent experiments. Heparin (Leo Pharma AB, Box 404, SE- 20124 Malmö, Sweden) was added in different concentrations (range 1-100 IE/ml) to wounded monolayers with or without HGF. **Cell proliferation:** CCL-53.1 cells were cultured as described before at a density of 1x 10⁶ cells per well in a 24-well plate (Cell Counter Multisizer, KEBO Lab, Spånga, Sweden). After the confluence, cells were separated with non-enzymatic cell dissociation medium (Sigma-Aldrich). DNA synthesis was determined by measuring incorporation of ³H-thymidine. Isolated CCL-53.1 cells were inoculated on a 24-well culture plate and cultured with F-12K medium supplemented with 15% horse serum and 2.5% fetal bovine serum for 24 hours. The day after the medium was changed to fresh medium and HGF (5 ng/ml) was added to triplets of well and simultaneously 50 µl of [Methyl ³H] thymidine (Amersham Pharmacia Biotech Europé GmbH Tyskland) was added to each well. The cells were incubated 24 hours before the DNA-assay method, as described before (Betsholtz C and Westermark 1984), was performed.

**Migration assay:** Cells were cultured on sterile round cover glasses placed in the bottom of 24-well dishes until confluence, which were removed after 24 hours and placed upside down in petri dishes (Nuncolone) containing 5 ml fresh medium. HGF (5ng/ml) was added to dishes and incubated under night in an atmosphere of 5% CO₂ and 95% air at 37°C. The dishes were then examined by light microscope (Olympus, Tokyo) and the length of migration of cells around the glass, were measured at 7 locations. The median of 7 measurements has been used for calculation of the mean of median values of ten experiments for statistical analysis.

**Cytoskleton and confocal microscopy:** Cells were cultured on cover glass. Confluent monolayer was injured and incubated with 10 ng/ml HGF for 24 hours as described before. After fixation (4% Para formaldehyde in PBS for 30 minutes at rooms temperature), cells were washed in three times in KRG and one unit of Alexa 488-phalloidin (Molecular Probes, OR.) in 200 µl KRG was added to each glass with cells. Labelling was continued for 20 minutes at room temperature, protected from light, on a Bellydancer (Stovall, Life Science, INC, Greensbro, NC,USA). After labelling, the Alexa 488-phalloidin was removed and glasses were washed 3x5 minutes in KRG and mounted in ProLong (Molecular Probes, OR). Cells were studied in a Sarastro 2000 confocal laser scanning microscope (Molecular Dynamics, Sunnyvale, CA.) using the 488 nm wavelength of the argon ion laser for excitation and a x60 objective with N.A. 1.4. Horizontal x-y section series with a distance of 1.4 µm between the sections, as well as vertical x-z sections were collected. 3D look-through projections were made at different angels and the 90-degree projections were used for comparing heights of control and HGF-treated cells.

**Detection of C-met/HGF/SF receptor by Immunofluorescence:** Confluent monolayer was injured and incubated with 10 ng/ml HGF for 24 hours as described before. The day of experiment, cells were fixed with 4% Para formaldehyde in PBS for 30 minutes at rooms temperature, then thoroughly washed twice in PBS and to block non-specific binding of antibodies pre-incubated with blocking buffer consisting of 5% bovine serum albumin (BSA) in PBS for 1 hour at rooms temperature. The monolayer was then incubated with a rabbit polycolonal antibody against the extracellular domain of mouse HGF receptor (Sigma-Aldrich) (diluted 1:50 in blockings buffer) for 60 minutes at rooms temperature. The cells were washed as above and labelled with fluorescine isothiocyanate (FITC)-conjugated goat anti-rabbit IgG (1:50) (Sigma-Aldrich). After incubation for 60 minute at rooms temperature, cells were washed, mounted with 17% Gelvatol in PBS and examined by epifluorescence microscopy (Zeiss Axioskop).

**Example 1a: Cell proliferation:** The ratio of CPM (count per minute) in control and HGF treated wells was as followed: 2ng HGF/control =1.1 (0.95-1.20); 5ng HGF/control =1.1(1.04-1.18) and 15ng HGF/control = 1.15 (0.99-1.23). Cell proliferation in the epithelial cell line (CCL-53.1) was therefore not significantly increased by recombinant HGF as assessed by determination [³H]-thymidine uptake.

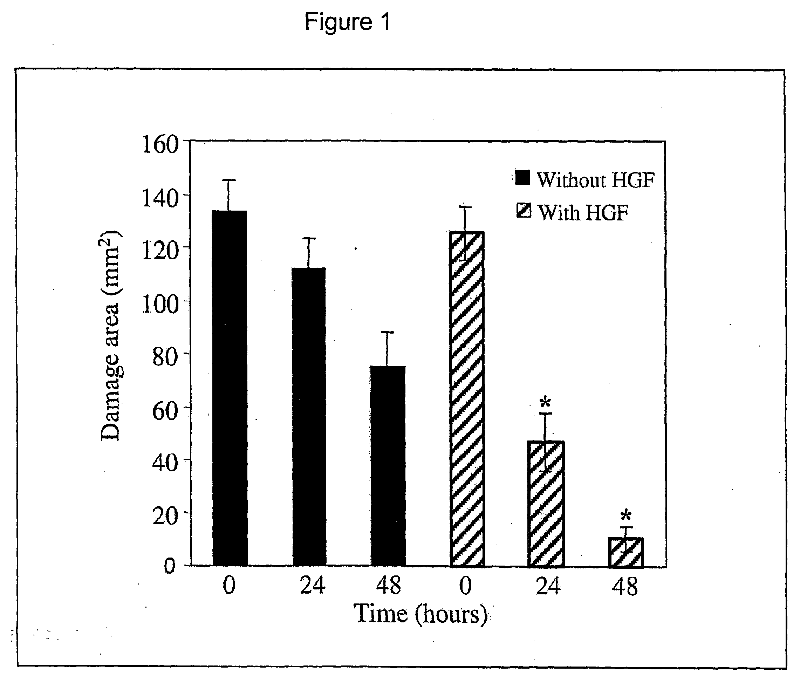

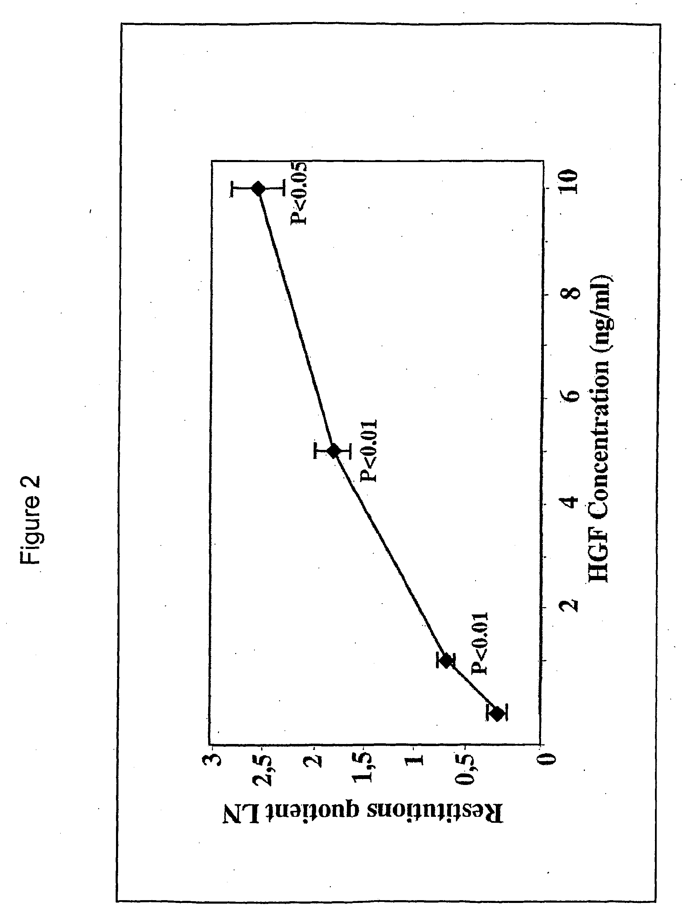

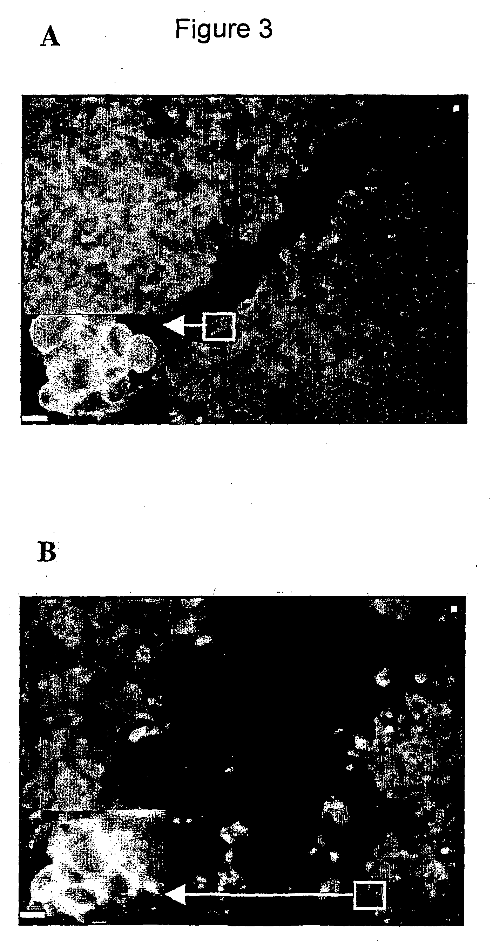

**Example 1b: Restitution of injured area:** The effect of HGF on restitution of injured mono layer was assessed using an *in-vitro* wounding model. The uncovered area decreased after 24 and 48 hours. The ratio of the measured bare area at the time of damage and after 24 hours by HGF was significantly higher (at the time of injury=125.25 ± 10.08 mm², after 24 hours = 46.73 ± 11.03 mm², ratio = 2.65, p<0.01 and after 48 hours 10.26 ± 4.76 mm², ratio = 11.91) compared to control (at the time of injury=133.77 ± 11.7mm² , after 24 hours = 112.23 ± 11.11 mm², ratio = 1.15 and after 48 hours 75 ± 13.17 mm², ratio=2.43) **(****Figure 1****).** There were no significant differences between ordinary and serum-deprived medium in restitution of injured cells after addition of HGF. Significant enhancement of epithelial restitution was observed at low HGF concentration, 1ng/ml. Increasing doses of HGF caused further increasing in restitutions rate up to 10ng/ml (p<0.01 between control and 1ng/ml, p<0.01 between 1ng/ml and 5ng/ml, p=0.05 between 5ng/ml and 10ng/ml) **(****Figure 2****).**
Adding FGF (1-15ng/mL), EGF (2-60ng/mL), PDGF (1-35 ng/ML), or FGF/KGF (1-15ng/mL) did not affect the restitution of injured cells significantly.
The c-Met receptor was visualised by Immunofluorescence in the wells. No significant differences were detected by this method in c-Met expression in the cells adjacent to the denuded area compared with the other cells distant from the wound after addition of HGF or in untreated controls **(****Figure 3****).**

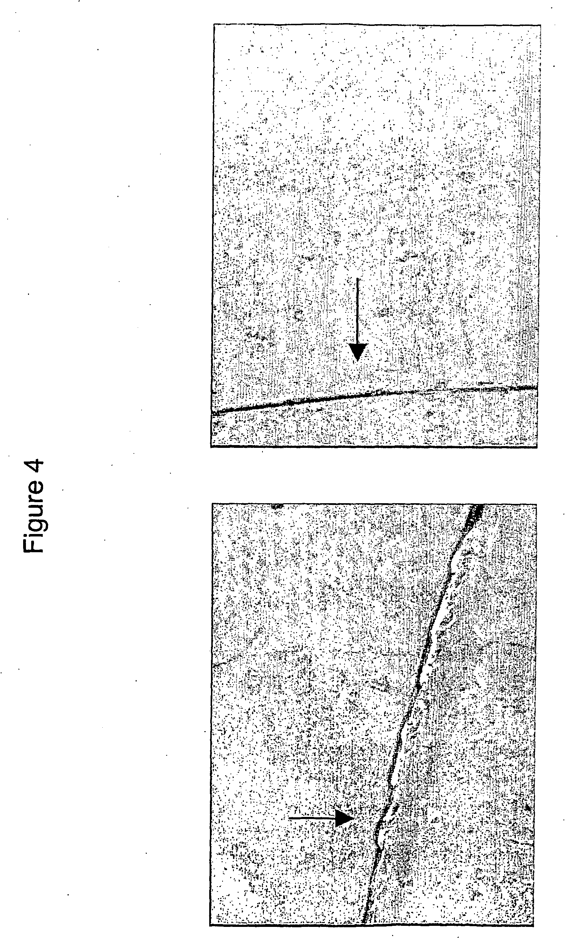

**Example 1c: Cell motility:** The distance of migration (mm) out of glass layer after addition of HGF was compared with untreated controls. An enhanced migration of epithelial cells after addition of HGF was shown (mean 10.6.± 1.66mm in HGF treated dishes and 1.2.± 0.51mm in controls) (p<0.01) **(****Figure 4****).**

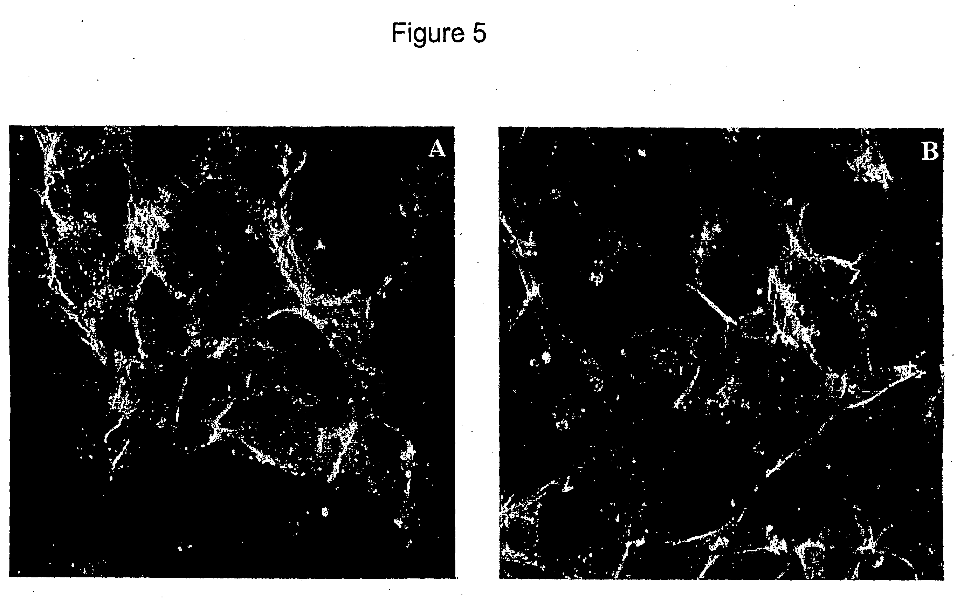

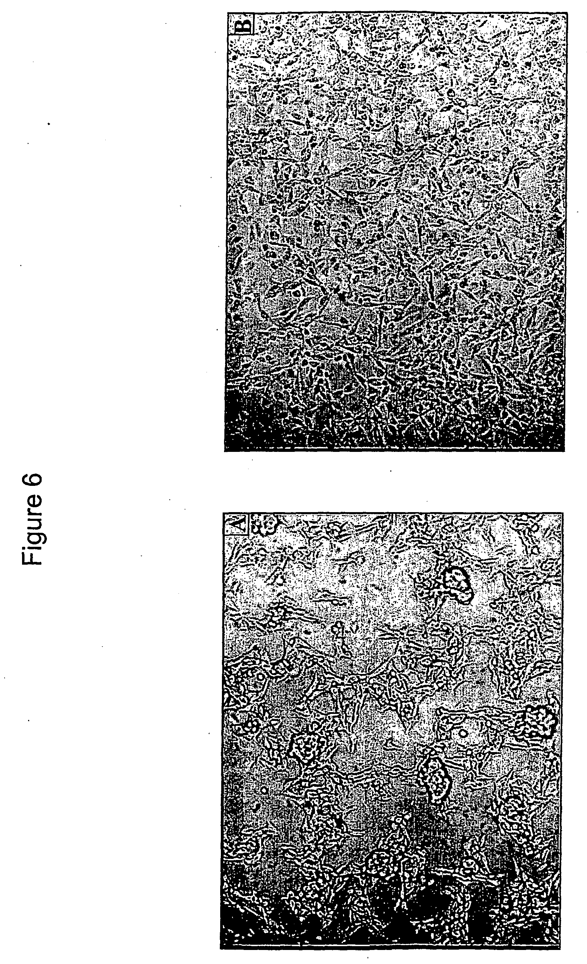

**Exempel 1d: Morphology and cytoskeleton:** The morphology of epithelial cells CCL-53.1 was different in HGF treated cells. In untreated wells the cells were gathered together in round dense glomerules with smaller intracellular spaces but had flattened out and spread morphology in HGF-treated wells. By 3-D reconstruction made by confocal microscopy technique, we found significant changes in actin structure after addition of HGF with less F-actin in the dense peripheral structures compared to untreated cells **(****Figure 5** **and** **6****).**

The examples above demonstrate how injured CC1-53.1 epithelial cells can be used to examine biological activity of HGF. Biologically active HGF dose-dependently enhances the restitution of injured CC1-53.1 epithelial cells after damage. These results might be explained partly by enhanced motogen effect, eventually as a result of weakened cytoskeleton structure after HGF treatment. The above findings were specific for active HGF and neither inactive HGF nor EGF, PDGF, FGF, KGF could induce identical effects on cells.

### EXAMPLE 2: The properties of biologically active hepatocyte growth factor (HGF) revealed by Surface Plasmon Resonance Method (Biacore).

The following materials and conditions were used.

**SPR measurements and ligand immobilization procedures.** SPR measurements were conducted at 760 nm in a fully automatic Biacore 2000 instrument and a semi-automatic Bicaore X instrument (Biacore AB, Uppsala, Sweden) equipped with four and two flow cells respectively. The flow cell temperature was 25 °C in all experiments. The sample surfaces used were carboxy-methylated dextran CM5 chips purchased from Biacore AB (Uppsala, Sweden). Coupling of ligands to the carboxylic acid groups of the dextran hydrogel was carried out by conventional carbodiimide chemistry using 200 mM EDC (N-ethyl-N'-(3-diethylaminopropyl) carbodiimide) and 50 mM NHS (N-hydroxysuccinimide). The activation time was 7 min, followed by a 2-7 min ligand injection. Deactivation of remaining active esters was performed by a 7 min injection of ethanolamine/hydrochloride at pH 8.5. A flow rate of 5 µl/min was used during all immobilizations. All ligands were diluted in 10 mM acetate buffer pH 4.5, i.e., below the protein isoelectric point, thus enhancing the electrostatic interactions between the dextran matrix and the ligands. The monoclonal anti-HGF (500 µg/ml) was diluted 1:10 and polyclonal anti-HGF. The recombinant Met proto-oncogene receptor (100 µg/ml) was diluted 1:5 and the HGF recombinant (5 ug/ml) 1:3. The contact time varied between two and seven minutes resulting in levels of immobilization between 8000 and 30 000 RU (response units). After deactivation, the surfaces were washed with five subsequent one-minute injections of 5 mM glycine buffer pH 2.0 with 1 M NaCl. One of the flow cells was used as a reference to monitor the response due to buffer and unspecific interactions as well as specific interaction of HGF to dextran. This flow cell was treated in the same way as the other during the immobilization procedure, but omitting the ligand immobilization step.

### Example 2a: Binding of HGF to dextran

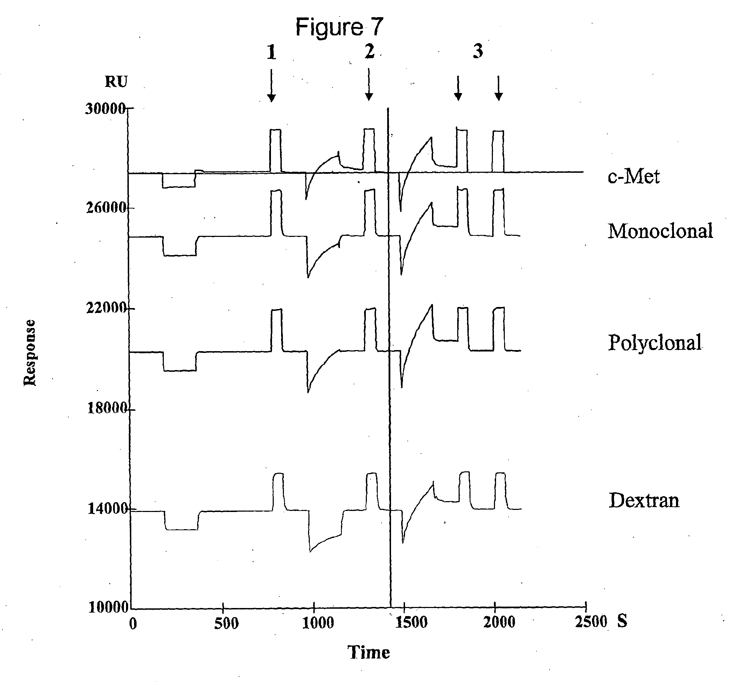

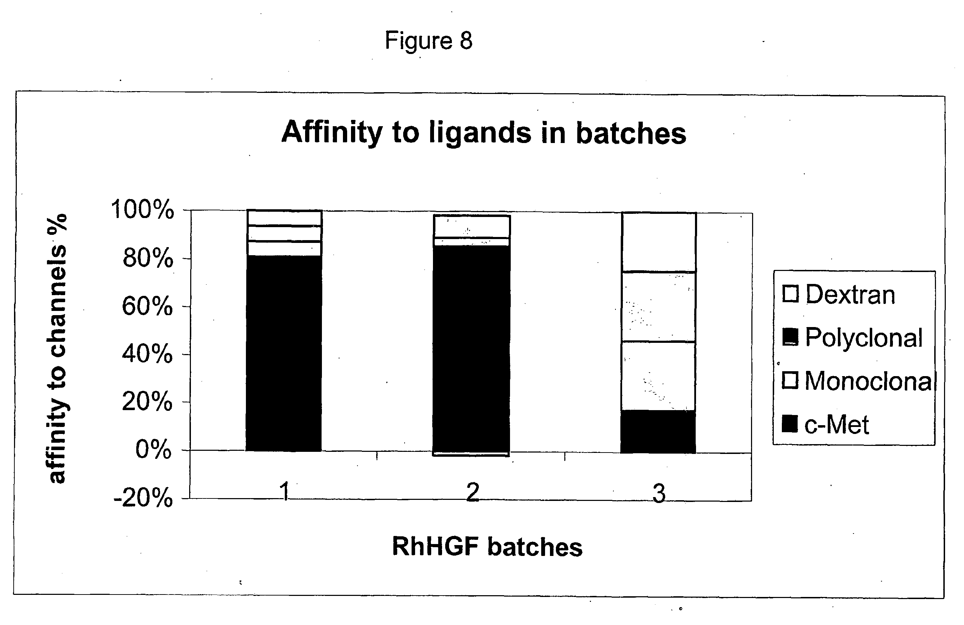

Biologically active HGF has a high affinity to dextran Addition of dextran (0.05%) to the samples resulted in a significant (p<0.05) decrease in signal responses in flow cells with immobilized monoclonal and polyclonal anti-HGF antibodies as well as in the dextran channel **(****Figure 7** **and** **8****).**

### Example 2b: Binding to ligands

Biologically active HGF has significantly higher signal response in the flow cells with immobilized monoclonal anti-HGF antibody (p=0.005) or c-Met receptor (p=0.02) compared to the biologically inactive HGF **(****Figure 7** **and** **8****).**

### Example 2c: Correlation with ELISA

Immunoreactive HGF was determined by ELISA using a commercially available kit (Quantikine HGF Immunoassy, R&D Systems Inc., Minneapolis, USA). When biologically active HGF was examined using determination with SPR, results correlated significantly with the levels measured by ELISA. ELISA and SPR results did not correlate when inactive HGF was used.

### Example 2d: Effects of altering the immobilization levels

The effect on signal responses due to the amount of monoclonal anti-HGF antibody immobilized was determined in different samples. Increasing the immobilization levels by increasing the contact time during immobilization (1, 5, and 10 minutes) caused a significantly higher response of all samples containing biologically active HGF. Altering the flow rate (5, 10, and 15 µl/min) did not have a significant effect on the response of the samples.

The above examples show that biologically active HGF seems to have a high affinity to dextran which might resemble such affinity to cell-surface heparan sulfate proteoglycan binding sites. These binding sites either overlap with the antibody-binding epitope or induce conformation shift in the HGF upon binding to the dextran, resulting in sterical hindrance and thus preventing binding to the antibody.

### EXAMPLE 3: The effects of local application of biologic active recombinant human hepatocyte growth factor (rHGF) to normal hairless skin in mice.

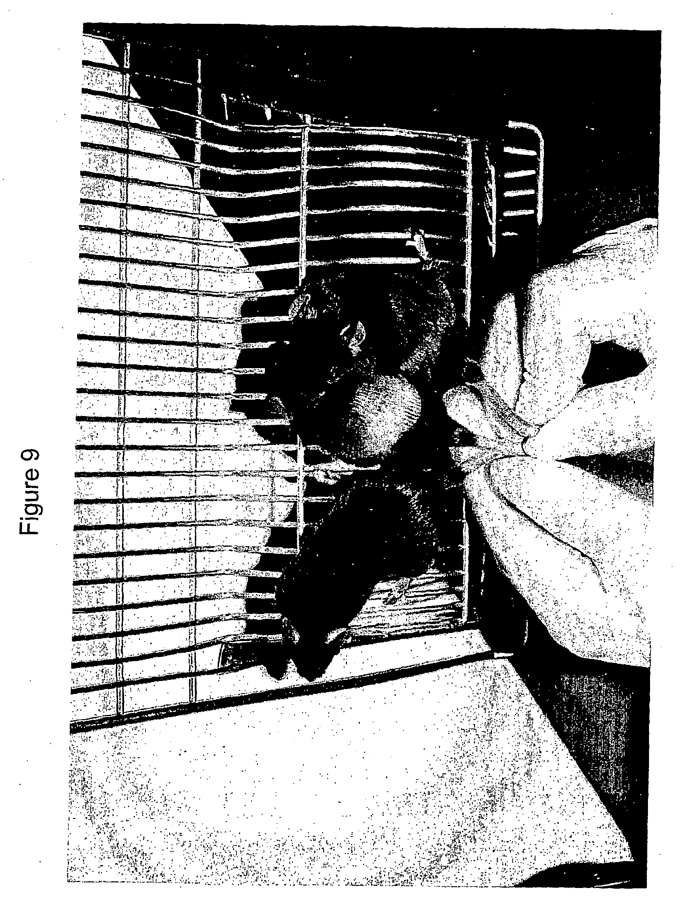

Eighteen mice (C57/B6 black, 14-16 weeks old and 21-29g weigh, all females) were shaved on their back causing two 1x1 cm patches on the right and left side. They were then divided into 3 groups. Group 1 (n=6) were treated subcutaneously on the right side by 0,70µg/kg (12-18ng/cm2 hairless skin) recombinant human hepatocyte growth factor (rHGF) daily in 5 days. The utilized HGF had been tested prior to study by ELISA, Surface Plasmon Resonance (SPR) method as well as by wound assay on CCL-53.1 cells. This form of HGF had high affinity to dextran and antibodies against HGF, determined by SPR and the results correlated to the concentration obtained by ELISA. The recombinant HGF caused migration of CCL-53.1 cells towards the injured area dose-dependently. This effect could be stopped by anti-HGF antibodies. Group 2 (n=6) received subcutaneously in 5 days 0,70µg/kg recombinant HGF that showed no affinity to dextran by SPR method and did not cause migration of CCL-53.1 cells in the wound assay. Group3 (n=6), received sterile physiologic sodium chloride subcutaneously in 5 days. This group served as the control group. None of the treated animals did show any signs of non-tolerance or diseases under or after course of treatment. Although some significant differences were not obviously seen in the hair growth under treatment we were surprised to see that the animals in Group 1 did show a full double-sided hair growth in 5 animals on day 12 **(****Figure 9****)**. The same process in Group 2 and Group 3 did take much a longer time (20-35 day). We conclude that local treatment of the normal, hairless skin in mice with the biologically active HGF with the appropriate dose hastens the hair growth and this local treatment is tolerated by animals without problem.

### EXAMPLE 4: Therapeutic effects of recombinant hepatocyte growth factor on chronic leg ulcers in patients with venous insufficiency - case reports

To evaluate the positive effects of hepatocyte growth factor on chronic leg ulcers observed during a recent pilot study, a double-blind placebo-controlled study on 20 patients using a commercial recombinant HGF in one week was started. The study was interrupted after inclusion of twelve patients when SDS-PAGE and western blot revealed that the protein used was defective. Immunohistochemistry staining of biopsies from ulcer area showed that HGF receptor (c-met) expression decreased non-significantly in the patients who had received the preparation containing commercial recombinant HGF (Group 1, n=6) compared to the group that received sodium chloride preparation (Group 2, n=6), that might indicate interaction of protein with c-met receptor. Enhanced capillary proliferation in Group 1 was observed but only in one case a dramatic clinical improvement was seen. Nevertheless, three cases in Group 1 and two in Group 2 were healed within 9 months. The other seven patients were offered treatment with biologically active recombinant HGF, and four (two in each group) accepted (Group 3). Improvement in the ulcer situation was observed in all of these initially unsuccessful cases within the first week of treatment. We concluded that biologically active HGF had positive effects on healing of chronic leg ulcers not attributed to placebo effects.

### EXAMPLE 5: HGF epitope mapping by SPR

### Example 5a: Antibody affinities.

By immobilizing several epitope specific antibodies against HGF (Santa Cruz) to the flow cells in SPR we have succeeded in determining a number of the properties of biologically active or inactive recombinant HGF.
1- The biologically active HGF, has a constant affinity to the different epitopes in the alfa and beta chains of HGF.
2- In the biologically inactive HGF, the affinity to flow cell immobilized by N-17 (antibody against a peptide mapping at the N-terminus of HGF alfa of human origin) was decreased or abolished.
   The affinity to H-170 (antibody raised against amino acid 1-170 of HGF beta of human origin) was decreased.
   The affinity to c-met receptor and H-145 (antibody against amino acid 32-176 HGF alfa of human origin) was maintained.

### Example 5b: HGF receptor binding epitopes

Using SPR we have succeeded in determining the epitopes that are important in binding of HGF to c-met receptor. We have shown that the epitopes of HGF that bind to H-145 (antibody against amino acid 32-176 HGF alfa of human origin) and C-20 (antibody raised against a peptide mapping at the C-terminus of HGF alfa of human origin) are participating when HGF binds to c-met receptor. The other epitopes that interact with N-17 ( antibody against a peptide mapping at the N-terminus of HGF alfa of human origin), N-19 ( antibody against a peptide mapping at the N-terminus of HGF beta of human origin) and H-170

(antibody raised against amino acid 1-170 of HGF beta of human origin) are still available during interaction of HGF with c-met receptor.

In the inactive HGF recombinants that we have tested, the receptor binding epitopes were intact but the domain interacting with N-17 from alfa chain and H-170 from beta chain were defective.

## Claims

1. Method for determination of biological activity of hepatocyte growth factor (HGF), wherein the biological activity of HGF is determined using affinity measurements and/or epitope mapping using a specific ligand to HGF, wherein said ligand is dextran.

2. Method according to claim 1 where determination of the biological activity of HGF is used for quality control of HGF after batch production, for example by recombinant techniques for the preparation of a pharmaceutical composition comprising biologically active HGF.

3. Method according to claim 1 where determination of the biological activity of HGF is used for evaluation of HGF preparations prior to administration to a patient in need of treatment with therapeutically active HGF.

4. Method according to claim 1 where determination of the biological activity of HGF is used for evaluation of activity of endogenous HGF in body tissue or fluids at the site of a lesion, such as in ulcer secrete, urine, feces, saliva, pleura in order to determine if application of exogenous therapeutically active HGF to the injured area is required.

5. Method according to claim 1 where determination of the biological activity of HGF is used for refining the diagnosis of various HGF related diseases.

## Patentansprüche

1. Verfahren zur Bestimmung der biologischen Aktivität von Hepatocytenwachstumsfaktor (HGF), wobei die biologische Aktivität von HGF durch die Verwendung von Affinitätsmessungen und/oder Epitopkartierung bestimmt wird, die einen spezifischen Liganden von HGF verwenden, wobei der Ligand Dextran ist.

2. Verfahren nach Anspruch 1, wobei die Bestimmung der biologischen Aktivität des HGF für die Qualitätskontrolle von HGF nach Produktion einer Charge, zum Beispiel durch rekombinante Techniken für die Herstellung einer pharmazeutischen Zusammensetzung, die biologisch aktiven HGF umfasst, verwendet wird.

3. Verfahren nach Anspruch 1, wobei die Bestimmung der biologischen Aktivität von HGF für die Beurteilung von HGF-Zusammensetzungen verwendet wird, bevor diese einem Patienten verabreicht werden, der eine Behandlung mit therapeutisch wirksamem HGF benötigt.

4. Verfahren nach Anspruch 1, wobei die Bestimmung der biologischen Aktivität von HGF für die Beurteilung der Aktivität von endogenem HGF in Körpergewebe oder Flüssigkeiten an der Stelle einer Läsion, wie etwa Ulcussekret, Urin, Fäzes, Speichel, Rippenfell, verwendet wird, um zu bestimmen, ob die Anwendung von exogenem therapeutisch wirksamem HGF an der Verletzungsstelle benötigt wird.

5. Verfahren nach Anspruch 1, wobei die Bestimmung der biologischen Aktivität von HGF verwendet wird, um die Diagnose von verschiedenen HGF-bezogenen Krankheiten weiter zu entwickeln.

## Revendications

1. Procédé de détermination de l'activité biologique du facteur de croissance des hépatocytes (HGF), dans lequel l'activité biologique du HGF est déterminée en utilisant des mesures d'affinité et/ou une cartographie des épitopes à l'aide d'un ligand spécifique du HGF, dans lequel ledit ligand est le dextrane.

2. Procédé selon la revendication 1, dans lequel la détermination de l'activité biologique du HGF est utilisée pour le contrôle qualité du HGF après une production discontinue, par exemple à l'aide de techniques de recombinaison, pour la préparation d'une composition pharmaceutique comprenant du HGF biologiquement actif.

3. Procédé selon la revendication 1, dans lequel la détermination de l'activité biologique du HGF est utilisée pour l'évaluation de préparations de HGF avant l'administration à un patient nécessitant un traitement avec du HGF thérapeutiquement actif.

4. Procédé selon la revendication 1, dans lequel la détermination de l'activité biologique du HGF est utilisée pour évaluer l'activité du HGF endogène dans un tissu cellulaire ou des fluides corporels au niveau du site d'une lésion, notamment dans des sécrétions ulcéreuses, l'urine, les selles, la salive, la plèvre, afin de déterminer si l'application du HGF exogène thérapeutiquement actif sur la région lésée est requise.

5. Procédé selon la revendication 1, dans lequel la détermination de l'activité biologique du HGF est utilisée pour affiner le diagnostic de plusieurs maladies associées au HGF.
